# EUROPEAN PATENT APPLICATION

(11) **EP 1 955 720 A1**
(43) Date of publication of application: **13.08.2008**
(21) Application number: 08250199.0
(22) Date of filing: 16.01.2008
(51) Int. Cl.: A61L 17/00, A61L 17/10, A61L 17/14

(54) **Surface eroding barbed sutures**

(30) Priority: 09.02.2007 US 673073
(71) Applicant: Tyco Healthcare Group, LP, North Haven, CT 06473 (US)
(72) Inventor: Stopek, Joshua, Yalesville, CT 06492 (US)
(74) Representative: Alcock, David

(57) **Abstract**

Barbed surgical sutures comprised of surface eroding materials are provided having increased degradation rates due to the formation of barbs and an increase in the surface area of the suture.

## Description

### RELATED PATENTS

This provisional application is related to U.S. Patent Application No. 11/556,002, the entire contents of which are incorporated herein by reference.

### TECHNICAL FIELD

The present disclosure relates to barbed sutures made of surface eroding materials having degradation rates adjusted to a desired strength and mass loss profile.

### BACKGROUND OF RELATED ART

Barbed sutures, which are generally made of the same materials as conventional sutures, offer several advantages for closing wounds compared with conventional sutures. A barbed suture includes an elongated body that has one or more spaced barbs, which project from the surface of the suture body along the body length. The barbs are arranged to allow passage of the barbed suture in one direction through tissue but resist movement of the barbed suture in the opposite direction. Thus, one advantage of barbed sutures has been the decreased time in closing wounds due to less knot tying by the surgeon.

Barbed sutures are known for use in cosmetic, laparoscopic and endoscopic procedures. Using barbed sutures enables the placement of tension in tissue with less slippage of the suture in the wound. Similar to a conventional suture, a barbed suture may be inserted into tissue using a surgical needle.

Depending on the tissue and wound type, patient health and corresponding healing rates, there is an optimal time needed for sutures to be present in the patient. Current sutures provide strength for a given period of time, however, suture mass is still present weeks after body no longer requires them to be. For example, a suture may provide strength for 10 days but is not completely absorbed by the body until 56 days. This foreign body may interfere with healing as it continues to degrade well after suture strength is needed. Therefore, it may be advantageous to incorporate surface eroding polymers in barbed sutures whose properties can provide necessary strength and appropriate mass loss, enabling the wound healing process.

### SUMMARY

The present disclosure provides a suture which includes a surface eroding material; an elongated body with a proximal end and a distal end; at least one length of barbs projecting from the elongated body towards at least one end and forming an included angle of less than about 90 degrees between the barbs and the elongated body.

In embodiments, the surface eroding material is selected from the group consisting of polyhydroxybutyrates, polyhydroxy alkoanates, polyorthoesters, lactones, poly(alpha-hydroxy esters), valerolactone, N-trimethyl chitosan chloride, homopolymers thereof, copolymers thereof, and combinations thereof.

A method of closing a wound with the suture of the present disclosure is also provided. In alternative embodiments, a needle is affixed to the suture.

### BRIEF DESCRIPTION OF THE DRAWINGS

Various embodiments of the present disclosure will be described hereinbelow with reference to the figures wherein:
FIG. 1 is a perspective view of a barbed suture in accordance with the present disclosure attached to a needle; and
FIG. 2 is a perspective view of a bi-directional barbed suture in accordance with the present disclosure attached to a needle on each end.

### DETAILED DESCRIPTION

Described herein is a degradable surgical suture having barbs extending from the body of the suture. The suture may have both a proximal and distal end, with barbs projecting from the elongated body towards at least one end thereby forming an included angle of less than about 90 degrees between the barbs and the suture body. The materials utilized to form the suture are selected to provide the suture with desired mass loss profile, strength retention, and tensile properties. Additionally, the rate of hydrolysis and subsequent mass loss in the hydrolytic degradation of the suture may be altered by changing the surface area through the addition of barbs.

Depending, for example, on the necessary time for healing a wound, a specific rate of degradation of a suture may be desired. It is important to ensure that mass loss is proportional to strength loss, namely, that the suture strength remains present to support the wound, while still absorbing within a reasonable time. Depending on the suture and application, the mass loss and strength loss may typically be within 30 days of each other.

Utilizing surface eroding polymer and methods of the present disclosure, the mass loss profile of a barbed suture may be optimized so that the suture is completely degraded at the most appropriate time, which may be of importance in certain major surgeries such as cosmetic, laparoscopic and endoscopic procedures. For example, in embodiments, the formation of barbs on the suture may permit the use of a degradable material which normally has high tensile strength and a long period of degradation to form a suture having similar high tensile strength but an increased degradation rate.

The barbed suture of the present disclosure may be made of any suitable surface eroding polymers which include polyanhydrides and anhydride copolymers, for example and including 1,6-bis(p-carboxyphenoxy)hexane (CPH) as the hydrophobe and 1,8-bis-(p-carboxyphenoxy)-3,6-dioxaoctane (CPTEG) (or other oligomer polyethyleneglycol (PEG) containing units) as the hydrophilic component.

In yet other embodiments, a suture of the present disclosure may be made of polyhyrdoxybutyrates and polyhydroxy alkoanates, polyorthoesters, poly(alpha-hydroxy esters), valerolactone, N-trimethyl chitosan chloride, and dioxanone. Degradable lactones may incorporate the use of silicone oils/oligomers (hydroxyl terminated) as chemical initiator, or D5 cyclic monomer for copolymerization with lactone monomers. In addition, another embodiment includes using an anionic ring opening polymerization of cyclic lactone monomers with oligomeric/cyclic bisphenol A to prepare degradable phenylated carbonates (i.e., TMC with phenol).

Suitable lactone monomers may be selected from the group consisting of glycolide, lactide (I, d, dl, meso), dioxanone, ε-caprolactone, delta-valerolactone, beta-butyrolactone, epsilon-decalactone, 2,5-diketomorpholine, pivalolactone, alpha, alpha-diethylpropiolactone, ethylene carbonate, ethylene oxalate, 3-methyl-1,4-dioxane-2,5-dione, 3,3-diethyl-1,4-dioxan-2,5-dione, gamma-butyrolactone, 1,4-dioxepan-2-one, 1,5-dioxepan-2-one, 1,4-dioxan-2-one, 6,8-dioxabicycloctane-7-one, trimethylene carbonate and combinations thereof.

Sutures of the present disclosure may be of monofilament or multifilament braids. Fibers used for forming sutures of the present disclosure may be formed using any technique within the purview of those skilled in the art, such as, for example, extrusion, molding and/or solvent casting. In embodiments, the fibers can be extruded through an extruder unit of a conventional type, such as those disclosed in U.S. Pat. Nos. 6,063,105; 6,203,564; and 6,235,869, the entire contents of each of which are incorporated by reference herein.

In embodiments, the suture of the present disclosure may include a yam made of more than one fiber, which may contain multiple fibers of the same or different materials. Where the sutures are made of multiple fibers, the suture can be made using any known technique such as, for example, braiding, weaving or knitting. The fibers may also be combined to produce a non-woven suture. The filberts themselves may be drawn, oriented, crinkled, twisted, commingled or air entangled to form yarns as part of the suture forming process. In one embodiment a multifilament suture of the present disclosure can be produced by braiding.

Barbed sutures and placement methods suitable for use according to the present disclosure include those described in U.S. Patent Nos. 5,931,855, and 6,599,310, and U.S. Patent Application Publication Nos. 20030074023, 20030074023, 20040088003, 20040060409, and 20040060410, the entire disclosures of each of which are incorporated by reference herein.

Barbs may be formed on the surface of the body of a suture utilizing any method within the purview of one skilled in the art. Such methods include, but are not limited to, cutting, molding, and the like. In some embodiments, barbs may be formed by making with acute angular cuts directly into the suture body, with cut portions pushed outwardly and separated from the body of the suture. The depth of the barbs thus formed in the suture body may depend on the diameter of the suture material and the depth of the cut. In some embodiments, a suitable device for cutting a plurality of axially spaced barbs on the exterior of a suture filament may use a cutting bed, a cutting bed vise, a cutting template, and a blade assembly to perform the cutting. In operation, the cutting device has the ability to produce a plurality of axially spaced barbs in the same or random configuration and at different angles in relation to each other. Other suitable methods of cutting the barbs include the use of various manual methods including blades.

In embodiments, needles may be attached to sutures for easier penetration into tissue. In order to facilitate needle attachment to a suture of the present disclosure, conventional tipping agents can be applied to the braid. Two tipped ends of the suture may be desirable for attaching a needle to each end of the suture to provide a so-called double armed suture. The needle attachment can be made by any conventional method such as crimping, swaging, etc., including those described in U.S. Pat. Nos. 5,133,738; 5,226,912; and 5,569,302, the disclosures of which are incorporated by reference herein. Sutures may incorporate various sizes, lengths, diameters and needles the like, curved, straight, or otherwise. Additionally, sutures can be packaged in any number of desired pre-cut lengths and in pre-shaped curves.

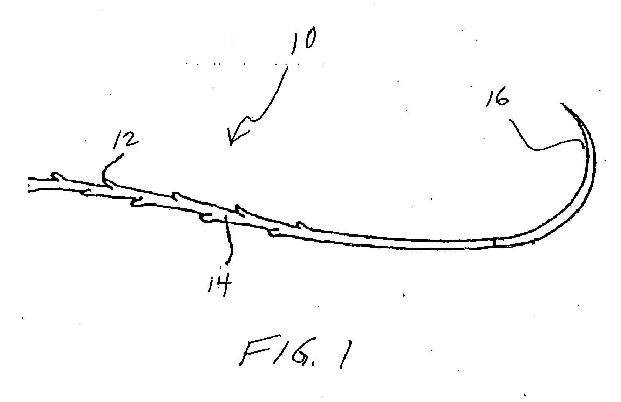

In embodiments, all of the barbs may be aligned to allow the suture to move through tissue in one direction and resist moving through tissue in the opposite direction. For example, referring to Fig. 1, the barbs 12 on a suture 10 may be formed into a single directional suture. The suture 10 includes an elongated body 14 and a plurality of barbs 12 extending from the periphery of the body. The barbs 12 are yieldable toward the body of suture 10. The barbs 12 permit movement of suture 10 through tissue in the direction of movement of a needle end 16 but are generally rigid in an opposite direction and prevent movement of suture 10 in a direction opposite the direction of movement of a needle end 16.

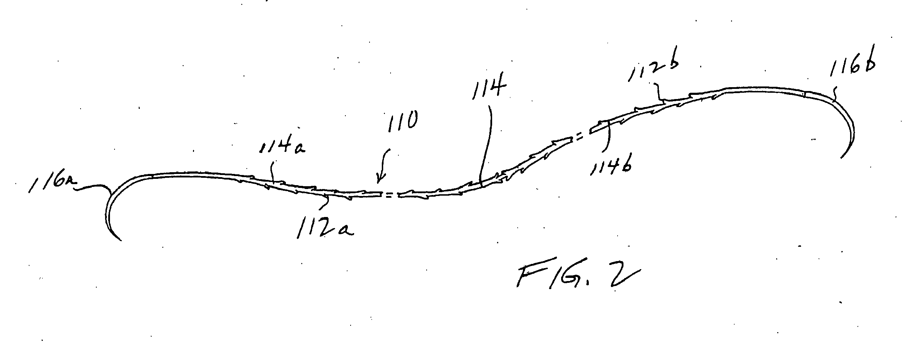

In other embodiments, the barbs may be aligned on a first portion of a length of a suture to allow movement of a first end of the suture through tissue in one direction, while barbs on a second portion of the length of the suture may be aligned to allow movement of the second end of the suture in an opposite direction. For example, as depicted in Fig. 2, a suture 110 may be bi-directional. Barbed suture 110 includes an elongated body 114 having two areas, body portion 114a and body portion 114b, distal first and second needle ends 116a and 116b for penetrating tissue, and a plurality of barbs 112a and 112b extending from the periphery of the body 114. Barbs 112a on a first portion of the body 114a between the first end of suture 110 and a first axial location on the suture body permit movement of suture 110 through the tissue in a direction of movement of first needle end 116a and prevent movement of suture 110 relative to the tissue in a direction opposite the direction of movement of the first needle end 116a. Barbs 112b on second portion of body 114b between a second needle end 116b of a suture 114 and a second axial location on the body which is less than the distance from a second needle end 116b to the first axial location permit movement of a suture 114 through the tissue in a direction of movement of a second needle end 116b and prevent movement of a suture 114 relative to the tissue in a direction opposite the direction of movement of the second needle end 116b.

The barbs can be arranged in any suitable pattern, for example, in a helical pattern. The number, configuration, spacing and surface area of the barbs can vary depending upon the tissue in which the suture is used, as well as the composition and geometry of the material utilized to form the suture.
Additionally, the proportions of the barbs may remain relatively constant while the overall length of the barbs, number, and spacing of the barbs may be determined by the tissue being connected.

The surface area of the barbs can also vary. For example, fuller-tipped barbs can be made of varying sizes designed for specific surgical applications. Larger or smaller barbs may be desired based on end use in specific tissues and procedures. In some embodiments, a combination of large and small barbs within the same structure may be beneficial, for example when a suture is used in tissue repair with differing layer structures. Use of the combination of large and small barbs with the same suture wherein barb sizes are customized for each tissue layer will ensure maximum anchoring properties. In embodiments a single directional suture as depicted in Figure 1 may have both large and small barbs; in other embodiments a bi-directional suture as depicted in Figure 2 may have both large and small barbs.

Barbed sutures fabricated from a degradable material in accordance with the present disclosure maintain their structural integrity after implantation (e.g., about 80% of original strength) for a predetermined period of time, depending on the characteristics of the particular copolymer used. Such characteristics include, for example, materials selection, monofilament versus multifilament in addition to processing conditions (e.g., rate of copolymerization reaction, temperature for reaction, pressure, etc.), and any post-treatment of the resulting sutures, i.e., drying, coating, sterilization, etc.

Barbed sutures of the present disclosure typically maintain their structural integrity, i.e., 80% of their original strength, after implantation for periods of time ranging approximately from about 3 to about 28 days, in embodiments from about 5 to about 21 days, in other embodiments from about 7 to about 14 days.

In accordance with the present disclosure, the formation of barbs on a surface eroding suture body may be utilized to increase the degradation time of a suture from about 1 % to about 25%, in embodiments from about 2% to about 20%, in other embodiments from about 3% to about 10%.

A bioactive agent may be impregnated within a polymer resin, combined during the extrusion process or applied to the surface thereof. In embodiments, a bioactive agent may also be included in a coating of such a suture. In some embodiments, the bioactive agent may be localized in the angle formed between the barb and the body of the suture, as described in U. S. Provisional Application No. 60/842,763 which is incorporated herein by reference.

Suitable bioactive agents include, for example, biocidal agents, antibiotics, antimicrobial agents, medicants, growth factors, anti-clotting agents, analgesics, anesthetics, anti-inflammatory agents, wound repair agents and the like, and combinations thereof.

Bioactive agents include substances which are beneficial to the patient and tend to promote the wound healing process. For example, a suture can be provided with a bioactive agent which will be released at the sutured site. For example, bioactive agents can be chosen for their antimicrobial properties, capability for promoting wound repair and/or tissue growth, or for specific indications such as thrombosis. In embodiments, combinations of such agents may be applied to or incorporated within a suture of the present disclosure.

The term "antimicrobial agent" as used herein includes an agent which helps the body destroy or resist pathogenic (disease-causing) microorganisms. An antimicrobial agent includes antibiotics, antiseptics, disinfectants and combinations thereof. Antimicrobial agents such as broad spectrum antibiotics (gentamicin sulfate, erythromycin or derivatized glycopeptides) which are slowly released into the tissue can be applied in this manner to aid in combating clinical and sub-clinical infections in a surgical or trauma wound site. In embodiments, suitable antimicrobial agents may be soluble in one or more solvents.

Classes of antibiotics that can be used as the antimicrobial agent include tetracyclines like minocycline; rifamycins like rifampin; macrolides like erythromycin; penicillins like nafcillin; cephalosporins like cefazolin; beta-lactam antibiotics like imipenem and aztreonam; aminoglycosides like gentamicin and TOBRAMYCIN^{®}; chloramphenicol; sulfonamides like sulfamethoxazole; glycopeptides like vancomycin; quinolones like ciprofloxacin; fusidic acid; trimethoprim; metronidazole; clindamycin; mupirocin; polyenes like amphotericin B; azoles like fluconazole; and beta-lactam inhibitors like sulbactam.

Examples of antiseptics and disinfectants which may be utilized as the antimicrobial agent include hexachlorophene; cationic biguanides like chlorhexidine and cyclohexidine; iodine and iodophores like povidone-iodine; halo-substituted phenolic compounds like PCMX (i.e., p-chloro-m-xylenol) and triclosan (i.e., 2,4,4'-trichloro-2'hydroxy-diphenylether); furan medical preparations like nitrofurantoin and nitrofurazone; methenamine; aldehydes like glutaraldehyde and formaldehyde; and alcohols. In some useful embodiments, at least one of the antimicrobial agents may be an antiseptic such as triclosan.

To promote wound repair and/or tissue growth, one or more bioactive agents known to achieve either or both of these objectives can also be incorporated in the suture as wound repair agents or tissue growth agents. Such materials include any of several human growth factors (HGFs), magainin, tissue or kidney plasminogen activator to cause thrombosis, superoxide dismutase to scavenge tissue-damaging free radicals, tumor necrosis factor for cancer therapy, colony stimulating factor, interferon, interleukin-2 or other lymphokines to enhance the immune system, combinations thereof, and so forth. Still further, surface eroding polymer drugs prepared from condensation polymerization of monomeric or multimeric drugs including, but not limited to non-steriodal anti-inflammatory drugs (NSAIDS) such as salicyclic acid and diflunisal.

Sutures in accordance with this disclosure can also include, for example, biologically acceptable plasticizers, antioxidants and colorants, which can be impregnated into the filament(s) utilized to form a suture of the present disclosure or included in a coating thereon.

As noted above, bioactive agents may be impregnated into the polymer resin or coated on the surface thereof. Bioactive agents may be applied onto a barbed suture of the present disclosure utilizing any method within the purview of one skilled in the art including, for example, dipping, spraying, vapor deposition, brushing, and the like. In embodiments the bioactive agent, such as an antimicrobial agent, may be combined with the resin and extruded.

In other embodiments, a barbed suture, or a portion thereof, may be coated with a biocompatible material which may impart desired handling characteristics. The addition of a coating should not, however, adversely affect the strength and tensile properties of the suture. Suitable coatings which may be utilized are within the purview of one skilled in the art and include, for example, biodegradable coatings such as those disclosed in U.S. Patent Publication No. 20040153125, the entire disclosure of which is incorporated by reference herein.

An "effective antimicrobial amount" of a given component is an amount at which the component hinders the growth of bacteria to diminish or avoid contamination of the wound site.

In embodiments, the antimicrobial degradable coating composition for biocompatible surgical implantable devices is inexpensive, biocompatible, and not subject to excessive diffusion. "Biocompatible" meaning, the ability of a material to perform within an appropriate host response in a specific application (DF Williams 1987), Appropriate host responses include resistance to blood clotting, resistance to bacterial colonization and normal uncomplicated healing.. It is contemplated that biocompatible objects may be associated with some clinically acceptable amounts of toxicity including irritation and/or other adverse reactions in certain individuals.

Such a coating may provide sutures with the combined desirable properties of improved handling characteristics, antimicrobial activity and strength loss which is proportional to mass loss.

In addition to the antimicrobial agents described above, in some embodiments the coating may include fatty acid metal salts which may impart antimicrobial characteristics to the suture.

Where a bioactive agent is included as part of a coating, the bioactive agent and coating components may be added to separate solvents, and the resulting solvent mixtures may then be combined to form a coating solution. In other embodiments, the bioactive agent and coating components may be combined together and then mixed with solvent to form a coating solution or any combination..

The coating can be applied to a suture by any suitable process, e.g., passing the suture through a solution of the coating mixture, past a brush or other coating solution applicator, or past one or more spray nozzles dispensing the suture coating solution. In embodiments, a mixture of methylene chloride, hexane and ethanol may be used as a solvent. The suture wetted with the coating solution may be optionally passed through or held in a drying oven for a time and at a temperature sufficient to vaporize and drive off the solvent. If desired, the suture coating composition can optionally contain additional bioactive agents or components described above, e.g., dyes, antibiotics, antiseptics, growth factors, anti-inflammatory agents, etc.

In embodiments, sutures of the present disclosure may be dyed in order to increase the visibility of the suture in the surgical field. Any dye suitable for incorporation in sutures can be used. Such dyes include, but are not limited to, carbon black, bone black, D&C Green No. 6, and D&C Violet No. 2.

The degradation rate of the resulting sutures may be affected by several factors including the character of the coating composition and the quantity applied. In some embodiments, it may be desirable to further treat the barbed sutures of the present disclosure to obtain the desired rate of degradation. For example, in some embodiments it may be desirable to heat the sutures of the present disclosure to obtain the desired rate of degradation. The heating of the suture may also remove monomers remaining in the polymers utilized to form the sutures. Suitable temperature for heating the sutures can be from about 100° C to about 160° C, in embodiments from about 120° C to about 143° C, for a period of time from about 2 hours to about 24 hours, in embodiments from about 8 hours to about 16 hours. In some embodiments, the heating may take place in a vacuum.

In other embodiments, the rate of degradation of the barbed sutures of the present disclosure may be controlled by exposing them to a plasma treatment, including a low-temperature gas plasma at a pressure substantially below atmospheric for a sufficient period of time. Such methods are within the purview of those skilled in the art and include, for example, the treatment disclosed in U.S. Patent No. 5,236,563, the entire disclosure of which is incorporated by reference herein. In embodiments, the surface treatment may be limited in time to treat the surface layer to a depth of from about 100 to about 1500 Angstroms.

In some cases a tubular insertion device (not shown) may be utilized to introduce a barbed suture in accordance with the present disclosure into tissue. Such a tubular insertion device may have a tubular body in which the barbed suture of the present disclosure is disposed, as well as a distal end and a proximal end. In use, the pointed end of a suture of the present disclosure may be pushed with the distal end of the tubular insertion device through skin, tissue, and the like at an insertion point. The pointed end of the suture and the distal end of the tubular insertion device are pushed through the tissue until reaching an endpoint. The proximal end of the tubular insertion device is then gripped and pulled to remove the insertion device, leaving the barbed suture in place.

Methods for repairing tissue with the sutures of the present disclosure are also provided. The sutures of the present disclosure may be utilized in any cosmetic endoscopic or laparoscopic methods. In addition, sutures of the present disclosure may be utilized to attach one tissue to another including, but not limited to, wound closure. As used herein, the term "wound" means a surgical incision, cut, laceration, severed tissue or accidental wound in human skin or other bodily tissue, or other condition where suturing, stapling, or the use of another tissue connecting device might be required. As used herein, the term "tissue" includes tissues such as skin, bone, muscle, organs, and other soft tissue such as tendons, ligaments and muscle.

In embodiments, sutures of the present disclosure may be held in place without the need for knots. In such cases, tissue located over a suture of the present disclosure placed in vivo may be physically manipulated or massaged into a desired position to enhance the holding of tissue in the desired position. In embodiments, the physical manipulation of tissue located over a suture of the present disclosure may enhance the release of any medicinal agent located on the suture, including any medicinal agent found in the angle between a barb and the body of a suture of the present disclosure. In other embodiments, the "S" or "J" stitching through tissue will activate the barbs, catching tissue and creating mechanical forces preventing the suture from moving backwards and holding without a knot. The term "activate" means causing the barbs to project outwards from suture.

For example, when the suture is used in tissue to repair a wound, the suture is passed through tissue at each of the sides of the wound. The point at one end of the suture, or in some embodiments, the needle, is inserted into a first side of a wound such that the point or needle pierces the tissue and the barbs on the end portion of the suture corresponding to the one end yield toward the body to facilitate movement of the suture through the tissue in the direction of insertion. The other end of the suture may also be inserted into a side of the wound and advanced through the tissue in like manner. The sides or faces of the wound are then moved together along the suture portions within the tissue to close the wound. The barbs of the suture grasp the surrounding tissue on each side of the wound and maintain the edges of the wound in position during healing. The leading ends of the suture protruding from the tissue are then cut and discarded. In one embodiment, ends of the suture in the tissue are made to lie below the surface of the skin by first depressing the skin immediately around the ends and severing the suture body closely against the skin. The skin will rise to cover the ends of the suture.

While the above description contains many specifics, these specifics should not be construed as limitations on the scope of the disclosure, but merely as exemplifications of embodiments thereof. Those skilled in the art will envision many other possibilities within the scope and spirit of the disclosure as defined by the claims appended hereto.

## Claims

1. A suture comprising:
a surface eroding material;
an elongated body with a proximal end and a distal end;
at least one length of barbs projecting from the elongated body towards at least one end and forming an included angle of less than about 90 degrees between the barbs and the elongated body.

2. The suture of claim 1, wherein the surface eroding material is selected from the group consisting of polyhydroxybutyrates, polyhydroxy alkoanates, polyorthoesters, lactones, poly(alpha-hydroxy esters), valerolactone, N-trimethyl chitosan chloride, homopolymers thereof, copolymers thereof, and combinations thereof.

3. The suture of claim 2, wherein the polyanhydride and anhydride copolymers comprise 1,6-bis(p-carboxyphenoxy)hexane and 1,8-bis-(p-carboxyphenoxy)-3,6-dioxaoctane copolymer.

4. The suture of claim 1, wherein the surface eroding material is selected from the group of polysalicyclic acid, and polyduflunisal.

5. The suture of claim 1, wherein the suture comprises a monofilament suture.

6. The suture of claim 1, wherein the suture comprises a multifilament suture.

7. The suture of claim 1, wherein the suture is affixed to a needle.

8. The suture of claim 1, further comprising a coating including a bioabsorbable polymer on at least a portion of the suture.

9. The suture of claim 8, wherein the coating includes a bioactive agent or drug.

10. The suture of claim 9, wherein the bioactive agent comprises biocidal agents, antibiotics, antimicrobial agents; medicants, growth factors, anti-clotting agents, analgesics, anesthetics, anti-inflammatory agents, wound repair agents and the like, and combinations thereof.

11. A method comprising closing a wound with a suture of claim 1.

12. A method comprising closing a wound with a suture of claim 1 affixed to a needle.

13. The method of claim 12, wherein the suture is advanced through tissue within a tubular insertion device.
